(19) Europäisches Patentamt
European Patent Office
Office européen des brevets

(11) EP 3 148 989 B1

(12) # FASCICULE DE BREVET EUROPEEN

(45) Date de publication et mention de la délivrance du brevet:
**25.04.2018 Bulletin 2018/17**

(21) Numéro de dépôt: **15732778.4**

(22) Date de dépôt: **20.05.2015**

(51) Int Cl.:
***C07D 407/14*** (2006.01) ***C08G 59/26*** (2006.01)
***C09D 163/00*** (2006.01) ***C09J 163/00*** (2006.01)

(86) Numéro de dépôt international:
**PCT/FR2015/051322**

(87) Numéro de publication internationale:
**WO 2015/181470 (03.12.2015 Gazette 2015/48)**

(54) **PROCÉDÉ DE FABRICATION DE COMPOSITIONS DE GLYCIDYL ETHERS FURANIQUES, COMPOSITIONS OBTENUES ET LEURS UTILISATIONS**

VERFAHREN ZUR HERSTELLUNG VON ZUSAMMENSETZUNGEN VON FURANGLYCIDYLETHERN, HERGESTELLTE ZUSAMMENSETZUNGEN UND VERWENDUNGEN DAVON

METHOD FOR PRODUCING COMPOSITIONS OF FURAN GLYCIDYL ETHERS, COMPOSITIONS PRODUCED AND USES OF SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.05.2014 FR 1454847**

(43) Date de publication de la demande:
**05.04.2017 Bulletin 2017/14**

(73) Titulaire: **Roquette Frères**
**62136 Lestrem (FR)**

(72) Inventeurs:
- **BUFFE, Clothilde**
  **59160 Lomme (FR)**
- **IBERT, Mathias**
  **59930 La Chapelle D'armentieres (FR)**

(74) Mandataire: **Cabinet Plasseraud**
**66, rue de la Chaussée d'Antin**
**75440 Paris Cedex 09 (FR)**

(56) Documents cités:
**US-A- 3 025 307 US-A1- 2012 220 742**

- **FENGSHUO HU ET AL: "Synthesis and Characterization of Thermosetting Furan-Based Epoxy Systems", MACROMOLECULES, vol. 47, no. 10, 9 mai 2014 (2014-05-09), pages 3332-3342, XP055137514, ISSN: 0024-9297, DOI: 10.1021/ma500687t cité dans la demande**
- **Fengshuo Hu ET AL: "Supporting Information: Synthesis and Characterization of Thermosetting Furan-Based Epoxy Systems", Macromolecules, vol. 47, no. 10 9 mai 2014 (2014-05-09), pages 1-9, XP055137620, Extrait de l'Internet: URL:http://pubs.acs.org/doi/suppl/10.1021/ma500687t/suppl_file/ma500687t_si_001.pdf [extrait le 2014-09-02]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention a pour principal objet un procédé de fabrication d'une composition de glycidyl éthers synthétisés à partir de dérivés furaniques (on parlera plus simplement dans la suite de la Demande de « glycidyl éthers furaniques ») dont une des originalités repose sur une distillation azéotropique conduite sous pression réduite et sans ajout de catalyseur. De tels produits sont utilisés pour fabriquer des résines époxy, dans le but de former un réseau macromoléculaire tridimensionnel.

Avec les compositions fabriquées selon l'invention, la densité de réticulation du réseau est augmentée ce qui permet d'obtenir un matériau plus résistant à la fois chimiquement et mécaniquement et présentant une température de transition vitreuse (Tg) plus élevée, par rapport aux mêmes matériaux obtenus avec les compositions de glycidyl éthers furaniques synthétisées à pression atmosphérique selon l'art antérieur.

En outre, les compositions de glycidyl éther furaniques fabriquées selon l'invention conduisent à des résines qui présentent une bien meilleure résistance à l'eau que leurs homologues fabriquées avec les compositions de dérivés furaniques synthétisées à pression atmosphérique selon l'art antérieur. Les compositions de glycidyl éther furaniques ainsi fabriquées peuvent être utilisées dans la fabrication de matériaux composites, de revêtements ou encore d'adhésifs.

Le glycidyl éther de Bisphenol A (BADGE ou DGEBA), de formule (I), est un composé chimique utilisé comme agent réticulant dans la fabrication des résines epoxy. Ce produit figure aujourd'hui sur la liste des cancérogènes du groupe 3 du CIRC (Centre International de Recherche sur le Cancer), c'est-à-dire qu'il s'agit d'une substance inclassable quant à sa cancérogénicité pour l'homme.

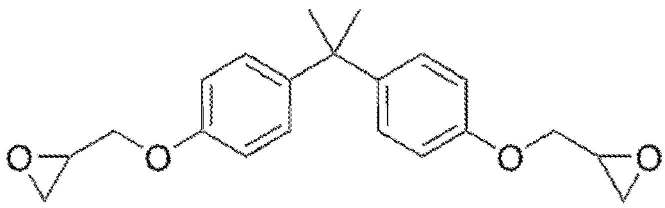

(I)

Or, le DGEBA est notamment utilisé comme additif dans les revêtements pour certaines boîtes de conserve. Du DGEBA libre peut donc se retrouver dans le contenu de ces boîtes, ce qui alimente de nombreuses questions au sujet de sa cancérogénicité (« Détermination of Bisphenol A diglycidyl ether and its hydrolysis products in canned oily foods from the Austrian market », Z. Lebensm. Unters. Forsch. A 208 (1999) pp. 208-211).

L'état de la technique fournit déjà un certain nombre de molécules, issues d'une « chimie verte » (non aliénée à des matières premières pétro-sourcées), dont les structures (formules II, II' et II" ci-dessous) miment de très près celle du DGEBA.

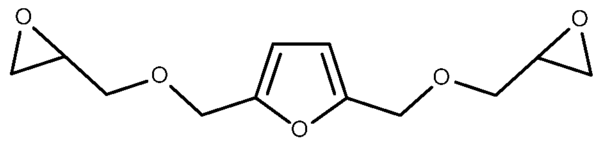

(II)

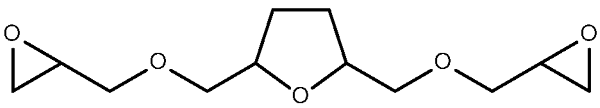

(II')

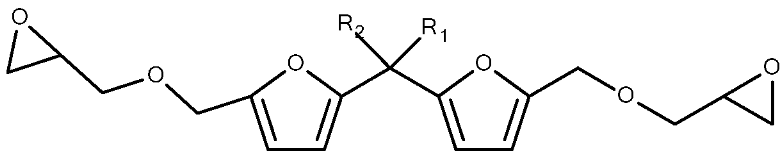

avec R1 et R2 pouvant être un hydrogène et/ou un groupement alkyl

(II'')

Ces composés, qui appartiennent à la classe plus générale des glycidyl éthers furaniques, sont aujourd'hui largement connus et décrits dans la littérature, de même que leur procédé de synthèse, qui font notamment intervenir respectivement les diols furaniques suivants : le DHMF (2,5-di(hydroxyméthyl)furane) et le DHMTHF (2,5-di(hydroxyméthyl)tétrahydrofurane) dont les structures sont données ci-après (formules III et III').

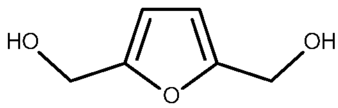

(III)

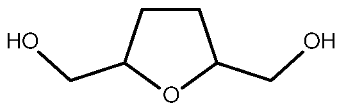

(III')

Le document US 2012 220742 décrit un procédé de synthèse en une seule étape par mise en contact d'un dérivé furanique avec de l'épichlorhydrine. La réaction a lieu à pression atmosphérique, à une température voisine de 50 °C, avec mise en oeuvre d'un catalyseur de transfert de phase et en présence d'un système solvant biphasique THF/Eau. Le dérivé époxy est alors récupéré par extraction liquide-liquide eau/acétate d'éthyle puis par purification sur colonne.

Le document US 3,025,307 décrit une procédé de synthèse en 2 étapes et à pression atmosphérique : tout d'abord par mise en contact du dérivé furanique avec de l'épichlorhydrine en présence d'un acide de Lewis, puis par introduction de soude en présence de solvant (THF typiquement).

Le document WO 2011 030991 divulgue quant à lui une méthode de synthèse en milieu solvant (THF) par mise en contact entre la soude, l'épichlorhydrine et le dérivé furanique à 50 °C environ, puis par purification sur colonne.

Plus récemment, l'article « Synthesis and Characterization of Thermosetting Furan-Based Epoxy Systems » (Macromolecules, Article ASAP, DOI: 10.1021/ma500687t, Publication Date (Web): May 9, 2014), décrit une synthèse en 2 étapes à pression atmosphérique. La première étape se fait en présence d'un catalyseur de transfert de phase, puis une solution aqueuse d'hydroxyde de sodium est introduite. Le produit est récupéré par extraction liquide-liquide eau/diethyl ether puis purifié sur colonne.

Ces procédés visent en fait à obtenir une composition (par opposition à un produit pur) contenant notamment des dérivés mono et difonctionnels. Or, seuls les dérivés difonctionnels participent à la formation du réseau macromoléculaire tridimensionnel au cours de la fabrication de la résine, notamment en présence de durcisseurs type amines. Ce sont donc ces dérivés difonctionnels qu'on cherchera à privilégier au détriment des produits monofonctionnels.

De la même manière, on veillera à limiter la teneur en oligomère (IV) et (IV') afin d'obtenir un réseau tridimensionnel ayant une densité de réticulation plus élevée. En effet, plus n est grand, plus la distance entre 2 fonctions réactives est grande et donc plus la distance entre chaque noeud de réticulation sera grande. Une densité de noeud importante permet d'obtenir un matériau plus résistant à la fois chimiquement et mécaniquement et ayant une température de transition vitreuse (Tg) plus élevée. Cette dernière propriété est fondamentale, pour des applications où le matériau synthétisé doit servir d'emballage à des produits « chauds », comme notamment des liquides. En effet, au-delà de la température de transition vitreuse, le matériau passe à l'état caoutchouteux, ses propriétés mécaniques sont alors changées : il devient plus souple et déformable.

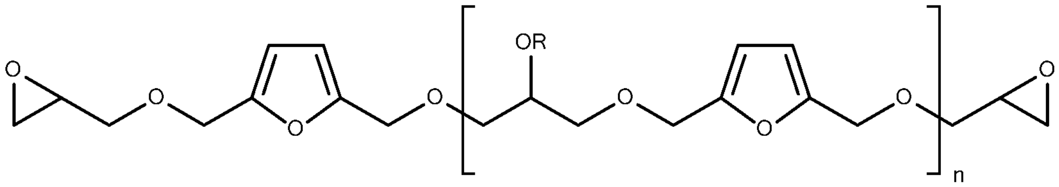

(IV)

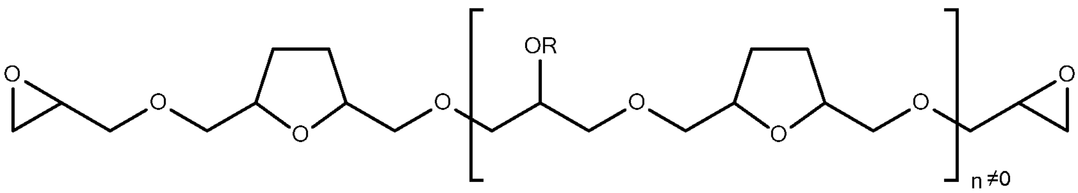

(IV')

La présence d'oligomères et/ou de dérivés monofonctionnels peut être directement reliée à l'équivalent d'époxy en poids, défini comme la masse de résine contenant un équivalent de fonction glycidyle. Par exemple, le diglycidyléther obtenu à partir du DHMF (formule II) qui a un poids moléculaire de 240 g / mol et qui contient 2 fonctions glycidyle, possède un équivalent époxy de 120 g / eq. Plus l'équivalent d'époxy en poids est élevé, plus la teneur en oligomère et/ou en dérivé monofonctionnel est élevée : on recherchera donc à minimiser cet équivalent d'époxy.
Aucun des documents de l'état de la technique précité qui visent des procédés de préparation de glycidyl éthers furaniques ne s'intéresse précisément à ce problème complexe qui consiste à augmenter la sélectivité vis-à-vis des dérivés difonctionnels au détriment des dérivés monofonctionnels et des oligomères. En outre, les documents de l'art antérieur précités mettent tous en oeuvre des solvants organiques autres que l'eau, solvants dont on cherche aujourd'hui à minimiser voire à proscrire l'usage.
Poursuivant ses recherches à travers de très nombreux travaux, la société Demanderesse est parvenue à mettre au point un procédé de fabrication de compositions glycidyl éthers furaniques, présentant en outre les avantages suivants :

- ledit procédé n'a recours qu'à l'eau en terme de solvant ;
- il ne met pas en oeuvre de catalyseurs, qui sont souvent des produits dangereux et/ou difficiles à éliminer et/ou à recycler (par exemple des halogénures, sulfates ou hydrogénosulfates de tétra-alkylammonium) ;
- l'équivalent d'époxy des compositions fabriquées s'avère avantageusement plus faible que celui des compositions de l'art antérieur ;
- la température de transition vitreuse d'une résine fabriquée avec les compositions de l'invention est avantageusement plus élevée que celle mesurée pour une résine fabriquée avec les compositions de l'art antérieur.

Une des originalités du procédé de l'invention est la conduite de la réaction entre le dérivé furanique (DHMF ou DHMTHF) et l'halogénure organique plus précisément l'épihalohydrine sous pression réduite, de manière à réaliser une distillation azéotropique. Ainsi, un premier objet de l'invention consiste en un procédé de fabrication d'une composition de glycidyl éthers furaniques de formule (II) ou (II'),

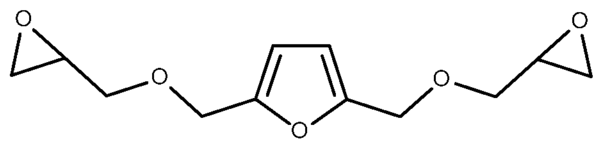

(II)

ou

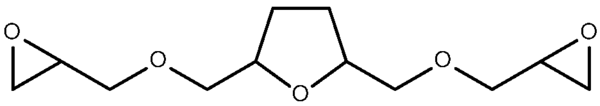

(II')

comprenant les étapes suivantes :

a) mettre en contact le DHMF ou le DHMTHF avec un halogénure organique qui est l'épichlorhydrine,
b) placer le mélange ainsi obtenu sous vide de manière à obtenir une dépression comprise 200 et 400 mbars,
c) chauffer le mélange sous vide à une température comprise entre 50°C et 120°C et réaliser ainsi une distillation azéotropique,
d) ajouter ensuite au dit mélange un réactif basique pendant une durée comprise entre 1 heure et 10 heures et poursuivre alors la distillation azéotropique,
e) récupérer la composition après une étape de filtration, concentration du filtrat et éventuellement une étape de purification.

La première étape du procédé selon l'invention (étape a) consiste donc à mettre en contact le DHMF ou le DHMTHF avec l'épichlorhydrine.

Cet halogénure organique est préférentiellement introduit en excès par rapport aux fonctions hydroxyles du DHMF et du DHMTHF. Ainsi, pour 1 mole de DHMF ou de DHMTHF on introduira préférentiellement entre 2 et 20 moles d'halogénure organique et plus préférentiellement environ 10 moles.

Cette première étape est effectuée dans tout dispositif bien connu de l'homme du métier, permettant de réaliser la mise en contact entre 2 réactifs chimiques, et étant muni d'organes de chauffage et d'agitation. Il peut s'agir, à titre d'exemple, d'un réacteur double enveloppe. Le dispositif en question doit également être équipé d'un organe permettant de réaliser un vide partiel et d'un organe permettant de conduire une distillation azéotropique, tel qu'un montage Dean-Stark inversé surmonté d'un réfrigérant.

Après cette première étape de mise en contact (étape a), on réalise ensuite un vide partiel dans le dispositif à l'aide d'une pompe à vide, la dépression correspondante étant comprise entre 200 et 400 mbars et préférentiellement entre 240 et 280 mbars (étape b). Au cours de la troisième étape du procédé de l'invention (étape c), on chauffe le mélange entre le DHMF ou le DHMTHF et l'halogénure organique à une température comprise entre 50 °C et 120 °C. Cette température est préférentiellement comprise entre 70 et 90 °C et plus préférentiellement entre 75 et 85 °C.

La température du fluide caloporteur circulant dans la double enveloppe du réacteur doit être réglée de manière à être au moins égale à la température d'ébullition de l'halogénure organique utilisé, de manière à commencer la distillation azéotropique. Au cours de cette première phase de distillation, ladite distillation ne concerne que l'halogénure organique : en d'autres termes, seule une partie de l'halogénure organique est éliminée par distillation. Par ailleurs, la température d'ébullition qu'il convient de prendre en compte est la température d'ébullition de l'halogénure organique sous la pression partielle qui règne dans le dispositif.

A titre d'exemple, l'épichlorhydrine présente une température d'ébullition de 116 °C à pression atmosphérique, cette température d'ébullition étant environ égale à 80 °C sous un vide partiel de 275 mbars. De manière pratique, on se placera à une température légèrement supérieure (environ 3 °C de plus) à la température d'ébullition pour l'halogénure organique considéré et pour la dépression imposée.

Au cours de la quatrième étape du procédé de l'invention (étape d), on ajoute alors au mélange DHMF ou DHMTHF / halogénure organique un réactif basique, pendant une durée comprise entre 1 heure et 10 heures, préférentiellement entre 1h et 6h et plus préférentiellement entre 2h et 4h.

La quantité de réactif basique est préférentiellement la quantité stoechiométrique par rapport au nombre de fonctions hydroxyles du DHMF ou du DHMTHF (par exemple : 2 moles de soude pour 1 mole de dérivé furanique). On peut néanmoins choisir de se placer en léger excès par rapport à cette stoechiométrie.

Le réactif basique est choisi parmi les hydroxydes de lithium, potassium, calcium et sodium éventuellement sous forme d'une solution aqueuse, et est, très préférentiellement, une solution aqueuse d'hydroxyde de sodium.

Dès l'introduction du réactif basique (étape d), il y a formation d'eau par réaction entre le DHMF ou le DHMTHF et l'halogénure organique, de même qu'il peut y avoir apport d'eau supplémentaire par introduction du réactif basique sous forme de solution aqueuse. La distillation concerne alors le mélange eau et halogénure organique, le premier étant éliminé et le second retournant dans le milieu réactionnel. Dans le cas d'un dispositif Dean-Stark : l'eau constitue la phase supérieure qui est éliminée, alors que l'halogénure en partie inférieure est retourné dans le milieu réactionnel.

La distillation azéotropique est poursuivie jusqu'à élimination complète de l'eau. Ainsi le milieu réactionnel est encore chauffé pendant une durée comprise entre 30 minutes et 1 heure après la fin de l'addition du réactif basique.

Le milieu réactionnel est enfin filtré afin d'éliminer les sels formés au cours de la réaction entre l'halogénure et le DHMF ou le DHMTHF, comme le chlorure de sodium dans le cas de l'épichlorhydrine. Les sels ainsi récupérés sont lavés une nouvelle fois avec de l'épichlorhydrine. Les eaux de lavages sont ajoutées au premier filtrat puis concentrées de manière à éliminer notamment l'épichlorhydrine. L'étape de concentration se fait par exemple par distillation sous vide, par exemple dans un dispositif type rotavapor et/ou évaporateur film raclée. Lors de cette étape de concentration, le produit brut ou la composition est chauffé progressivement jusqu'à 140 °C et la pression est diminuée jusqu'à 1 mbar.
Eventuellement, une étape supplémentaire de purification par distillation sous pression réduite (<1mbar) peut être réalisée au moyen d'un échangeur à surface raclée afin de séparer les oligomères des dérivés monofonctionnels. Cette étape est distincte de celle décrite dans le paragraphe précédent. Les compositions susceptibles d'être obtenues selon le procédé de l'invention peuvent être utilisées pour la fabrication de matériaux composites, de revêtements et d'adhésifs.
Ces compositions peuvent aussi être utilisées pour la synthèse de vinylester par réaction avec des acides (méth)acryliques. Ces monomères photoréticulables (vinylesters) pourront alors être utilisés pour la fabrication de résines dentaires, coque de bateau, revêtement de spécialité.
Les compositions peuvent être utilisées dans des réactions de polycondensation afin d'obtenir un réseau tridimensionnel et un matériau thermorigide.
Dans ce cas, elles peuvent être utilisées seules (réactions d'homopolymérisation) ou en association avec d'autres monomères (réactions de co-polymérisation).
Parmi les co-monomères on peut citer les autres dérivés époxy mais aussi des agents dit durcisseurs ou de réticulation tels que les amines, polyétheramines, polyamides, amidoamines, bases de Mannich, anhydrides, polyesters polycarboxyliques, les mercaptans, les résines phénoliques, les résines mélamine, urée et phénol-formaldéhyde. Des catalyseurs type acide de Lewis, amine tertiaire, imidazole peuvent également être ajoutés à la formulation afin d'initier et/ou accélérer la réticulation. Les réactions de réticulation se feront à une température allant de 5°C à 260°C.
Les matériaux, résines obtenues à partir des compositions de glycidyl éthers furaniques, fabriquées selon la présent invention, sont plus résistantes chimiquement et mécaniquement et présentent en outre une température de transition vitreuse (Tg) plus élevée, par rapport aux mêmes matériaux obtenus avec les compositions de glycidyl éthers furaniques selon l'art antérieur, comme démontré ci-après.

**EXEMPLES**

**Réactifs :**

DHMF : commercialisé par la société Pennakem
DHMTHF : obtenu après hydrogénation du DHMF (95 %, Pennakem) sur Ni de Raney à 110 °C et 70 bars, puis purification par distillation
Epichlorhydrine : commercialisé par la société Sigma-Aldrich
Bromure de tetraéthyl ammonium : commercialisé par la société Sigma-Aldrich
Bromure de tetrabutyl ammonium : commercialisé par la société Sigma-Aldrich

**Exemple 1 :** Essais selon l'art antérieur.

3 essais ont été menés :

- 2 (essais 1 et 2) au cours desquels on a fait réagir respectivement le DHMF et le DHMTHF et l'épichlorhydrine, avec ajout d'une solution aqueuse de soude, la distillation azéotropique étant conduite à pression atmosphérique ;
- 1 selon les conditions du document WO 2011 030991 précité.

Essai n°1

On introduit, dans un réacteur double enveloppe de 1 litre, muni d'un bain thermostaté à fluide caloporteur, d'un système d'agitation mécanique à pâle, d'un système de contrôle de la température du milieu réactionnel et d'un Dean Stark inversé surmonté d'un réfrigérant, 50 g de DHMF (0,39 mol, 1 équivalent molaire) puis 361,2 g d'épichlorhydrine (3,90 mol, 10 équivalents molaires).
On chauffe alors le mélange réactionnel jusqu'à 116°C (température d'ébullition de l'épichlorhydrine = 116 °C à pression atmosphérique) pendant 30 minutes.
On ajoute alors progressivement 125 g d'une solution aqueuse d'hydroxyde de sodium à 50 % (1,56 mol, 2 équivalents

molaires).
L'addition dure au total 1h40 ; la distillation azéotropique se poursuit et l'eau formée par réaction entre l'halogénure (en l'espèce l'épichlorhydrine) et le DHMF est éliminée.

On filtre sous vide le milieu réactionnel afin d'en éliminer le chlorure de sodium formé au cours du temps. On lave finalement les sels avec de l'épichlorhydrine qui est ensuite éliminé par évaporation sous pression réduite au rotavapor.

On obtient alors la composition de diglycidyléther furanique ou contenant majoritairement du diglycidyléther furanique sous forme d'un liquide (viscosité Brookfield à 25 °C de 442mPa.s), ayant un équivalent d'époxy de 189 g / équivalent.

Essai n°2

On a procédé de la même manière que pour l'essai n° 1, à la différence que la matière première utilisée, le dérivé furanique est le DHMTHF.
On obtient alors la composition de diglycidyléther furanique ou contenant majoritairement du diglycidyléther furanique sous forme d'un liquide (viscosité Brookfield à 25 °C de 69 mPa.s), ayant un équivalent d'époxy de 162g / équivalent.

Essai n°3

Cet essai est réalisé dans les conditions du document WO 2011 030991 exemple 1 et met donc en oeuvre un solvant organique autre que l'eau.

Pour ce faire, on introduit, dans un réacteur double enveloppe de 1 litre, muni d'un bain thermostaté à fluide caloporteur, d'un système d'agitation mécanique à pâle, d'un système de contrôle de la température du milieu réactionnel et d'un Dean Stark inversé surmonté d'un réfrigérant, 252,8 g d'épichlorhydrine (14 équivalents molaires) puis 187,4g d'une solution aqueuse d'hydroxyde de sodium à 50% (12 équivalents molaires) et 4,10g de bromure de tétrabutylammonium.

On ajoute progressivement une solution contenant 25g de DHMF (1 équivalent molaire) et 293mL de tétrahydrofurane (THF). Le milieu réactionnel est maintenu sous agitation à 50 °C durant 2h.

On réalise une extraction liquide-liquide eau-acétate d'éthyle. On extrait 2 fois la phase aqueuse avec de l'acétate d'éthyle. On sèche les phases organiques avec du sulfate de magnésium anhydre et on concentre au rotavapor sous pression réduite.

On obtient alors la composition de diglycidyléther furanique ou contenant majoritairement du diglycidyléther furanique sous forme d'un liquide ayant un équivalent d'époxy de 405 g/équivalent.

Le tableau 1 récapitule les conditions opératoires, et notamment :
- Le ou les solvants mis en oeuvre ;
- la quantité d'épichlorhydrine mise en oeuvre, exprimée en équivalent molaire d'épichlorhydrine par rapport au nombre de mole de dérivé furanique (Eq Mol EPI) ;
- la quantité d'hydroxyde de sodium mise en oeuvre, exprimée en équivalent molaire d'hydroxyde de sodium par rapport au nombre de mole de dérivé furanique (Eq Mol NaOH) ;
- le temps d'introduction de la soude (temps intro NaOH).

**Tableau 1**

| Essai n° | 1 | 2 | 3 |
|---|---|---|---|
| solvant | eau | eau | eau/THF/ acétate d'éthyle |
| Eq Mol EPI | 10 | 10 | 14 |
| Eq Mol NaOH | 2 | 2 | 12 |
| Temps intro NaOH | 3h35 | 3h32 | |
| Equivalent Epoxy (g/eq) | 189 | 162 | 405 |

**Exemple 2** : Essais selon l'invention

2 essais selon l'invention (essais 4 et 5) ont été réalisés au cours desquels on a fait réagir le DHMF ou le DHMTHF et l'épichlorhydrine avec ajout d'une solution aqueuse de soude, la distillation azéotropique étant conduite sous un vide partiel.

Essai n°4

On introduit dans un réacteur double enveloppe de 1 litre, muni d'un bain thermostaté à fluide caloporteur, muni d'un système d'agitation mécanique à pâle, d'un système de contrôle de la température du milieu réactionnel et d'un Dean Stark inversé surmonté d'un réfrigérant, 50 g de DHMF (0,39 mol, 1 équivalent molaire) puis 361,2 g d'épichlorhydrine (3,9 mol, 10 équivalents molaires).

Le système est amené à une pression de 275 mbar relatif. On chauffe alors le mélange réactionnel jusqu'à 80 °C (température d'ébullition = 80 °C à 275 mbar) pendant 30 minutes avant de débuter l'addition contrôlée de 125 g d'une solution aqueuse d'hydroxyde de sodium à 50% (1,56 mol, 2 équivalents molaires). L'addition dure au total 3 h 35. L'eau est éliminée en continu par distillation azéotropique.

On filtre sous vide le milieu réactionnel afin d'en éliminer le chlorure de sodium formé au cours du temps. On lave les sels avec de l'épichlorhydrine qui est ensuite éliminé par évaporation sous pression réduite au rotavapor.

On obtient alors la composition de diglycidyléther furanique ou contenant majoritairement du diglycidyléther furanique sous forme d'un liquide limpide (viscosité Brookfield à 25 °C de 139 mPa.s) ayant un équivalent d'époxy de 137 g / équivalent.

Essai n°5

On a procédé de la même manière que pour l'essai n° 4, à la différence que la matière première utilisée est le DHMTHF.
On obtient alors la composition de diglycidyléther furanique ou contenant majoritairement du diglycidyléther furanique sous forme d'un liquide (viscosité Brookfield à 25 °C de 31 mPa.s), ayant un équivalent d'époxy de 139 g / équivalent.

**Tableau 2**

| Essai n° | 4 | 5 |
|---|---|---|
| Eq Mol EPI | 10 | 10 |
| Eq Mol NaOH | 2 | 2 |
| Temps intro NaOH | 3h35 | 3h35 |
| Pression (mBar) | 275 | 275 |
| Equivalent Epoxy (g/eq) | 137 | 139 |

Le tableau 3 indique la répartition déterminée par CPG (en % de surface) des différents constituants du produit final.
Dans tous les exemples de la présente demande, l'analyse CPG est réalisée sur une colonne capillaire de type DB1 (30m x 0,32 mm, épaisseur de film de 0,25μm). La quantification des espèces consiste à calculer la proportion relative des aires des pics du chromatogramme, le % de chaque espèce (x) étant égal à l'aire du pic de l'espèce (x) divisé par la somme de l'aire de tous les pics.

**Tableau 3**

| Essai n° | 1 | 4 |
|---|---|---|
| | Hors invention | Selon l'invention |
| DHMF | 0,9% | 0% |
| monoglycidylether DH MF | 26,3% | 3,8% |
| diglycidylether DHMF | 27% | 79,6% |
| monoglycidylether di-DHMF | 4% | 0% |
| diglycidylether di-DHMF | 5,1% | 6,4% |
| autres | 36,7% | 10,2% |
| % diglycidylether DHMF / (mono+diglycidylether DHMF) | 51% | 95,4% |

La comparaison entre les essais 1 et 4 démontre que le procédé selon l'invention permet d'obtenir des compositions dans lesquelles la proportion de diglycidyléther furanique par rapport au monoglycidylether furanique est considérablement plus importante.

**Exemple 3** :Réalisation de résines à partir de compositions fabriquées selon l'invention ou selon l'art antérieur.

Des résines époxy à partir des compositions précédentes et en présence d'un durcisseur de type amine (l'isophorone diamine) ont été préparées.
La quantité d'isophorone diamine introduite est calculée de manière à ce que le rapport du nombre de groupement -NH sur le nombre de groupement époxy soit égal à 1.

L'isophorone diamine est disponible sous la marque Vestamid® IPD par Evonik. L'équivalent de groupement -NH en poids est de 42,5g/eq. La formule utilisée pour calculer les mises en oeuvre de diamine est la suivante :

$$m\ (isophorone\ diamine) = \frac{mepoxy\ \times 42{,}5}{Equivalent\ epoxy}$$

A titre d'exemple, voici comment on a procédé pour l'essai 6. 5,06 g du produit obtenu à l'essai n°1 sont mélangés à température ambiante avec 1,1589 g d'isophorone diamine pendant 1 minute. Le mélange, homogène et s'écoulant à température ambiante, est placé dans un moule en silicone (L=43mm, l=20mm). La réticulation est effectuée en étuve pendant 1 heure à 80 °C et 2h à 180 °C.

On obtient alors un matériau solide à température ambiante et ayant une température de transition vitreuse (Tg) de 27 C. La température de transition vitreuse est mesurée par DSC au deuxième passage d'une rampe de température de -100 à 200 °C à 10 °C/min.

En outre, on a également déterminée, par double pesée avant et après immersion de chaque composition dans de l'eau pendant 24 heures, le pourcentage en poids d'eau absorbée.

Le tableau 4 récapitule les résultats obtenus suivant les compositions de glycidyl éthers furaniques utilisées.

**Tableau 4**

| Essai n° | 6 | 7 | 8 | 9 | 10 |
|---|---|---|---|---|---|
| Matière première | DHMF | DHMTHF | DHMF | DHMF | DHMTHF |
| Référence de l'essai correspondant au glycidyléther furanique utilisé | 1 (hors invention) | 2 (hors invention) | 3 (hors l'invention) | 4 (selon l'invention) | 5 (selon l'invention) |
| Equivalent d'époxy (g/eq) | 189 | 162 | 403 | 137 | 139 |
| Tg (°C) | 27 | 20 | 20 | 69 | 45 |
| % eau absorbée | 6,1 | 22,2 | 43,3 | 0,6 | 16,7 |

Pour une même matière première de départ, on note une nette diminution de l'équivalent d'époxy et, en parallèle, une forte augmentation de la température de transition de même qu'une diminution de la reprise en eau et ce, pour les essais réalisés avec les compositions fabriquées selon l'invention en comparaison avec les essais avec les compositions de l'art antérieur.

## Revendications

1. Procédé de fabrication d'une composition de glycidyl éthers furaniques de formule (II) ou (II'),

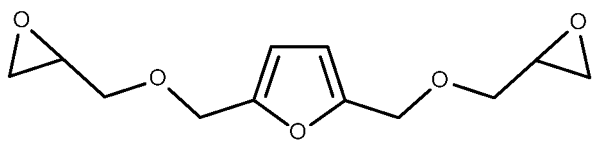

(II)

ou

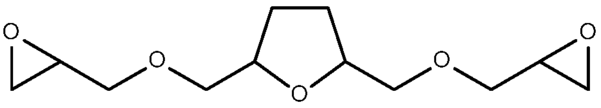

(II')

comprenant les étapes suivantes :

a) mettre en contact le 2,5-di(hydroxyméthyl)furane (DHMF) ou le 2,5-di(hydroxyméthyl)tétrahydrofurane (DHMTHF) avec l'épichlorhydrine,
b) placer le mélange ainsi obtenu sous vide de manière à obtenir une dépression comprise entre 200 et 400 mbars,
c) chauffer le mélange sous vide à une température comprise entre 50 °C et 120 °C et réaliser ainsi une distillation azéotropique,
d) ajouter ensuite au dit mélange un réactif basique pendant une durée comprise entre 1 heure et 10 heures et poursuivre alors la distillation azéotropique,
e) récupérer la composition après une étape de filtration, concentration du filtrat et éventuellement une étape de purification.

**2.** Procédé selon la revendication précédente, **caractérisé en ce que** l'épichlorhydrine est introduite en excès par rapport aux fonctions hydroxyles du DHMF et du DHMTHF.

**3.** Procédé selon l'une des revendications précédentes, **caractérisé en ce que** la dépression au cours de l'étape b) est comprise entre 240 et 280 mbars.

**4.** Procédé selon l'une des revendications précédentes, **caractérisé en ce que** la température au cours de l'étape c) est comprise entre 70 et 90 °C et plus préférentiellement entre 75 et 85 °C.

**5.** Procédé selon l'une des revendications précédentes, **caractérisé en ce que** la durée au cours de l'étape d) est comprise entre 1 h et 6 h et plus préférentiellement entre 2 h et 4 h.

**6.** Procédé selon l'une des revendications précédentes, **caractérisé en ce que** le réactif basique est choisi parmi les hydroxydes de lithium, potassium, calcium et sodium éventuellement sous forme d'une solution aqueuse, et est, très préférentiellement, une solution aqueuse d'hydroxyde de sodium.

## Patentansprüche

**1.** Verfahren zur Herstellung einer Zusammensetzung von Furanglycidylethern der Formel (II) oder (II'),

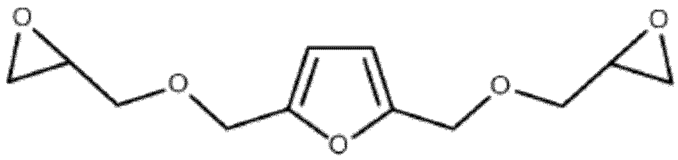

(II)

oder

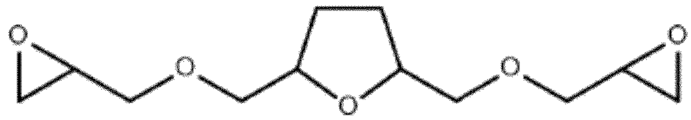

(II')

umfassend die folgenden Schritte:

a) Inkontaktbringen von 2,5-Di(hydroxymethyl)furan (DHMF) oder 2,5-Di(hydroxymethyl)tetrahydrofuran (DHMTHF) mit Epichlorhydrin,
b) unter Vakuum Setzen der so erhaltenen Mischung in einer Weise, dass ein Unterdruck von zwischen 200 bis 400 mbar erhalten wird,
c) Erhitzen der unter Vakuum gesetzten Mischung bei einer Temperatur von zwischen 50 °C und 120 °C und derart Bewirken einer azeotropen Destillation,
d) dann Zugeben eines basischen Reagenz zu der genannten Mischung während eines Zeitraums von zwischen 1 und 10 Stunden und dann Fortsetzen der azeotropen Destillation,
e) Gewinnen der Zusammensetzung nach einem Filtrationsschritt, Konzentration des Filtrats und gegebenenfalls einem Reinigungsschritt.

**2.** Verfahren gemäß dem vorhergehenden Anspruch,
**dadurch gekennzeichnet, dass**
das Epichlorhydrin im Überschuss bezogen auf die Hydroxylfunktionalitäten des DHMF und des DHMTHF zugesetzt wird.

**3.** Verfahren gemäß einem der vorhergehenden Ansprüche,
**dadurch gekennzeichnet, dass**
der Unterdruck in Schritt b) zwischen 240 und 280 mbar beträgt.

**4.** Verfahren gemäß einem der vorhergehenden Ansprüche,
**dadurch gekennzeichnet, dass**
die Temperatur in Schritt c) zwischen 70 und 90 °C und mehr bevorzugt zwischen 75 und 85 °C umfasst.

**5.** Verfahren gemäß einem der vorhergehenden Ansprüche,
**dadurch gekennzeichnet, dass**
die Dauer von Schritt d) zwischen 1 und 6 Stunden und mehr bevorzugt zwischen 2 und 4 Stunden umfasst.

**6.** Verfahren gemäß einem der vorhergehenden Ansprüche,
**dadurch gekennzeichnet, dass**
das basische Reagenz ausgewählt ist aus den Hydroxiden von Lithium, Kalium, Kalzium und Natrium, gegebenenfalls in Form einer wässrigen Lösung und ist, besonders bevorzugt, eine wässrige Lösung von Natriumhydroxid.

## Claims

**1.** A process for producing a composition of furan glycidyl ethers of formula (II) or (II'):

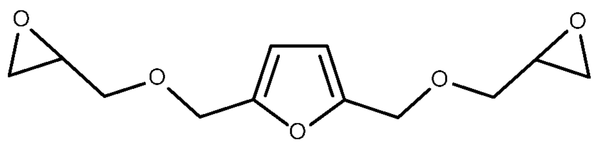

(II)

or

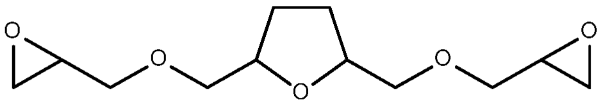

(II')

comprising the following steps:

a) bringing the 2,5-di(hydroxymethyl)furan (DHMF) or the 2,5-di(hydroxymethyl)tetrahydrofuran (DHMTHF) into contact with epichlorohydrin,
b) placing the mixture thus obtained under vacuum so as to obtain a low pressure of between 200 and 400 mbar,
c) heating the mixture under vacuum at a temperature of between 50 °C and 120 °C and thus carrying out an azeotropic distillation,
d) then adding to said mixture a basic reagent over a period of between 1 hour and 10 hours and then continuing the azeotropic distillation,
e) recovering the composition after a filtration step, concentration of the filtrate and optionally a purification step.

**2.** The process as claimed in claim 1, **characterized in that** the organic halide is introduced in excess relative to the hydroxyl functions of the DHMF and of the DHMTHF.

**3.** The process as claimed in one of the preceding claims, **characterized in that** the low pressure during step b) is between 240 and 280 mbar.

**4.** The process as claimed in one of the preceding claims, **characterized in that** the temperature during step c) is between 70 and 90 °C and more preferentially between 75 and 85 °C.

**5.** The process as claimed in one of the preceding claims, **characterized in that** the duration during step d) is between 1 h and 6 h and more preferentially between 2 h and 4 h.

**6.** The process as claimed in one of the preceding claims, **characterized in that** the basic reagent is chosen from lithium hydroxide, potassium hydroxide, calcium hydroxide and sodium hydroxide, optionally in the form of an aqueous solution, and is very preferentially an aqueous sodium hydroxide solution.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2012220742 A
- US 3025307 A
- WO 2011030991 A


### Littérature non-brevet citée dans la description


- **Z. LEBENSM.** Détermination of Bisphenol A diglycidyl ether and its hydrolysis products in canned oily foods from the Austrian market. *Unters. Forsch. A,* 1999, vol. 208, 208-211
- Synthesis and Characterization of Thermosetting Furan-Based Epoxy Systems. *Macromolecules,* 09 Mai 2014